Europäisches Patentamt

European Patent Office

Office européen des brevets

(19)

(11) Veröffentlichungsnummer: **0 180 859 B1**

## (12) EUROPÄISCHE PATENTSCHRIFT

(45) Veröffentlichungstag der Patentschrift: **10.06.92**

(51) Int. Cl.⁵: **C12N 9/72**, A61K 37/54, A61L 2/00

(21) Anmeldenummer: **85113501.2**

(22) Anmeldetag: **24.10.85**

Die Akte enthält technische Angaben, die nach dem Eingang der Anmeldung eingereicht wurden und die nicht in dieser Patentschrift enthalten sind.

(54) **Verfahren zur Reinigung sowie Pasteurisierung von Urokinase.**

(30) Priorität: **02.11.84 DE 3439980**

(43) Veröffentlichungstag der Anmeldung:
**14.05.86 Patentblatt 86/20**

(45) Bekanntmachung des Hinweises auf die Patenterteilung:
**10.06.92 Patentblatt 92/24**

(84) Benannte Vertragsstaaten:
**AT BE CH DE FR GB IT LI LU NL SE**

(56) Entgegenhaltungen:
**AT-B- 367 635**
**FR-A- 1 209 859**
**GB-A- 1 068 817**
**US-A- 4 169 764**

**CHEMICAL ABSTRACTS, Band 96, Nr. 23, 07 Juni 1982, Columbus, OH (US); L.LIU et al., Seite 303, Nr. 195614r***

(73) Patentinhaber: **BEHRINGWERKE Aktiengesellschaft**
**Postfach 1140**
**W-3550 Marburg 1(DE)**

(72) Erfinder: **Pâques, Eric, Dr.**
**Schmiedeacker 18**
**W-3550 Marburg(DE)**

(74) Vertreter: **Klein, Otto, Dr. et al**
**Hoechst AG Zentrale Patentabteilung Postfach 80 03 20**
**W-6230 Frankturt am Main 80(DE)**

EP 0 180 859 B1

## Beschreibung

Die Erfindung betrifft ein Verfahren zur Reinigung sowie Pasteurisierung von Urokinase. Eine nach diesem Verfahren gewonnene Urokinase kann als Thrombolytikum verwendet werden.

Plasminogen wird unter dem Einfluß von Katalysatoren in Plasmin umgewandelt. Zu den Katalysatoren dieser Reaktion zählt Urokinase. Urokinase ist beispielsweise in Spuren im Urin des Menschen zu finden. Die Anwendung von Urokinase zur Thrombolyse ist bekannt. Es ist auch bereits bekannt, daß die handelsüblichen Urokinase-Präparate häufig aus einer Mischung einer hoch- (54.000 M.W.) und einer niedermolekularer (33.000 M.W.) Form der Urokinase bestehen. Die hochmolekulare Urokinase (HMW-UK) entspricht der nativen Form des Moleküles, während die niedermolekulare Form (LMW-UK) ein proteolytisches Abbauprodukt der HMW-UK darstellt.

Für die therapeutische Anwendung wird die HMW-UK vorgezogen, weil sie der natürlichen Form entspricht. Ob die Umwandlung in die niedermolekulare Form durch Autokatalyse oder durch kontaminierende Proteasen bewirkt wird, ist ungeklärt (J. Biochem. 90 (225-232) 1981; Thromb. Res. 23 (541-547) 1981).

Es sind bereits verschiedene Methoden zur Isolierung und Reinigung von Urokinase beschrieben worden. Diese Verfahren führen vorwiegend zur Herstellung von Urokinase-Präparaten, welche durch ein ungünstiges Verhältnis von HMW- zu LMW-UK gekennzeichnet sind.

In DE-OS 28 15 853 ist eine Methode für die Gewinnung der HMW-Urokinase beschrieben. Die Ausbeute bei der Anwendung dieses Verfahrens ist, verursacht durch die Entfernung der LMW-Anteile, sehr gering, auch weil der Abbau der HMW-Urokinase nicht verhindert wird.

Die vorliegende Erfindung hat sich die Aufgabe gestellt, eine Urokinase-Präparation mit einem hohen Anteil an HMW-Urokinase, wie er im Urin zu finden ist, und hoher Stabilität herzustellen.

Es wurde überraschenderweise gefunden, daß durch Bindung der Urokinase aus einer Lösung an einen Kationenaustauscher und eine Stufen-Gradienten-Elution Urokinase mit guter Ausbeute von begleitenden Proteinen abgetrennt werden kann und dieser Reinigungsvorgang die Stabilität der HMW-Urokinase wesentlich erhöht. Es wurde weiterhin überraschenderweise gefunden, daß der Abbau der HMW-Urokinase während einer Hitzebehandlung zur Inaktivierung von Viren durch Verdünnen der Urokinaselösung auf eine Konzentration von 10.000 bis 100.000 IU/ml oder Einstellen auf einen pH-Wert kleiner als 6, vorzugsweise von 4 bis 5,95, verhindert werden kann.

Diese drei Verfahrensweisen können kombiniert werden.

Gegenstand der Erfindung ist somit ein Verfahren zur Reinigung von Urokinase, worin fremde Stoffe mit proteolytischer Aktivität entfernt werden, und das die Herstellung eines Produktes von hoher Stabilität in guter Ausbeute ermöglicht.

Gegenstand der Erfindung ist weiterhin ein Verfahren für die Pasteurisierung einer Lösung von Urokinase ohne wesentliche Verringerung des HMW-UK-Anteils und der Urokinase-Aktivität.

Gegenstand der Erfindung ist besonders ein Verfahren zur Reinigung von Urokinase, dadurch gekennzeichnet, daß eine Lösung von Urokinase mit einem Kationenaustauscher in Kontakt gebracht wird, der beladene Austauscher durch eine Stufen-Elution von Verunreinigungen mit proteolytischer Aktivität befreit und die Urokinase eluiert wird.

Als Ausgangslösung kann beispielsweise Urin eingesetzt werden.

Gegenstand der Erfindung ist auch ein Verfahren zur Inaktivierung von pathogenen Viren in einer Urokinaselösung durch eine Hitzebehandlung, dadurch gekennzeichnet, daß die Urokinaselösung nach Einstellung auf einen pH-Wert kleiner als 6, vorzugsweise von 4 bis 5,95, und eine Urokinase-Konzentration von 10.000 bis 100.000 IU/ml in Anwesenheit eines Mono- oder Disaccharids oder Zuckeralkohols und gegebenenfalls einer Aminosäure mindestens 1 Stunde, vorzugsweise 8 bis 20 Stunden, bei einer Temperatur von 40-90°C, vorzugsweise 55-65°C, erhitzt wird. Anschließend kann die Urokinaselösung konzentriert und sterilfiltriert werden. Die Konzentration des Saccharids ist vorzugsweise 400-600 g/l, die der Aminosäure 1-3 mol/l.

Gegenstand der Erfindung ist ferner die Anwendung eines auf diese Art hergestellten Präparates für therapeutische Zwecke, das insbesondere durch Injektion, Perfusion oder analoge Weise verabreicht werden kann.

Falls die Urokinase enthaltende wässrige Lösung störende Mengen Salze enthält, wird sie zweckmäßigerweise weitgehend von diesen befreit.

Weitere begleitende unerwünschte Proteine können gegebenenfalls beispielsweise mit Hilfe eines Anionenaustauschers entfernt werden.

Ein bevorzugtes Verfahren besteht darin, daß aus der die Urokinase enthaltenden Lösung gegebenenfalls Salze entfernt werden, gegebenenfalls die salzarme Lösung mit einem Anionenaustauscher zusammengebracht und der Austauscher abgetrennt, gegebenenfalls der Überstand oder andernfalls die entsalzte Lösung auf pH 5 bis 6, vorzugsweise 5,4-5,6, eingestellt und mit einem Kationenaustauscher, vorzugsweise CM-Cellulose, zusammengebracht wird. Der Kationenaustauscher wird mit einem Puffer, vorzugsweise bei pH 5,5, vorzugsweise mit einer Leitfähigkeit zwischen 5 und 10 mSi (20°C), gewaschen, und die Urokinase mit einem Puffer zwischen pH 5,5 und 9,5, vorzugsweise zwischen 6,5 und 9, vorzugsweise mit einer Leitfähigkeit zwischen 20 und 30 mSi (20°C) eluiert.

In einer besonders bevorzugten Ausführungsform kann man so verfahren, daß man die Urokinase enthaltende Lösung, beispielsweise Urin, gegebenenfalls entsalzt, gegebenenfalls durch Behandeln mit einem Anionenaustauscher von Verunreinigungen befreit, die Lösung auf pH 5,5 einstellt, mit CM-Cellulose versetzt, diese abtrennt und mit einem Puffer von pH 5,5 enthaltend 0,07 mol/l Natriumacetat und 0,5 mol/l Glycin (Leitfähigkeit: 0 bis 5 mSi; 20°C) wäscht, sie dann mit einem Puffer, enthaltend 0,15 mol/l Natriumacetat und 0,5 mol/l Glycin, pH 5,5 (Leitfähigkeit: 5 bis 10 mSi; 20°C) wäscht und dann die Urokinase mit einem Puffer bei pH 9 (Leitfähigkeit: 20 bis 30 mSi; 20°C) eluiert.

Man kann weiterhin so verfahren, daß man das Eluat auf einen pH-Wert von 5,5 einstellt und nach Zusatz von einem Mono- oder Oligosaccharid oder Zuckeralkohol, vorzugsweise 1 kg/l Saccharose, und gegebenenfalls einer wasserlöslichen Aminosäure, vorzugswseise 112 g/l Glycin, bei einer Endkonzentration zwischen 10.000 und 100.000 IU/ml Urokinase, vorzugsweise 50.000 IU/ml, 10 Stunden bei 60°C erhitzt, konzentriert und sterilfiltriert.

Ein auf die beschriebene Weise hergestelltes Präparat zeichnet sich dadurch aus, daß es das der Ausgangslösung entsprechende Verhältnis an HMW/LMW-Urokinase und eine hohe Reinheit aufweist und zugleich eine gute Stabilität besitzt.

Wenn beispielsweise ein Ausgangsmaterial mit einem Verhältnis von hochmolekularer zu niedermolekularer Urokinase von 70/30 eingesetzt wurde, dann wurde ein Produkt mit gleichem Verhältnis von HMW- zu LMW-UK in einer Ausbeute von 85% und in einer Reinheit von über 90% erhalten. Falls das Waschen des Kationenaustauschers unterblieb, war das HMW/LMW-Verhältnis des Produkts 55/45, die Ausbeute 90% und die Reinheit unter 60%. In diesem Fall war auch die Stabilität des Endproduktes geringer. Das Polymerenverhältnis verschob sich zugunsten des niedermolekularen Anteils beim Stehenlassen einer Lösung bei Raumtemperatur.

Das Verhältnis HMW/LMW-UK wurde nach Auftrennung der beiden Formen auf einer Mono-S-säule (FPLC-Pharmacia) durch Bestimmen der amidolytischen Aktivität ermittelt.

Ausbeute und Polymerenverhältnis des Endprodukts sind vom pH während des Erhitzungsschrittes abhängig. Vorteilhaft ist ein pH von etwa 5,5, wobei ein günstiges Verhältnis von hochmolekularer zu niedermolekularer Urokinase bei sehr guter Ausbeute erhalten wird.

Die Erfindung soll durch das nachstehende Beispiel näher erläutert werden.

Beispiel

1. Reinigung

Die urokinase-haltige Ausgangslösung wurde bei 4°C durch Gelfiltration von Salz befreit, der Durchlauf gesammelt und mit DEAE-Cellulose bei pH 8 von Verunreinigungen befreit. Der Durchlauf wurde mit 300 g/l Saccharose und 37,5 g/l Glycin versetzt, mit Salzsäure auf pH 5,5 eingestellt, bei 4°C mit CM-Cellulose versetzt, die mit einer 0,07 mol/l Natrium-Acetat und 0,5 mol/l Glycin enthaltenden Lösung mit pH 5,5 äquilibriert worden war, und 1 Stunde gerührt. Das Gel wurde trocken gesaugt, mit dem Äquilibrierpuffer gewaschen, in eine Säule gefüllt, mit einem Puffer, enthaltend 0,15 mol/l Natrium-Acetat und 0,5 mol/l Glycin, pH 5,5 (Leitfähigkeit: 7,8 - 8,1 mSi, 20°C), gewaschen, und anschließend die Urokinase mit einer Lösung, enthaltend 0,2 mol/l Natrium-Acetat und 0,5 mol/l Glycin, pH 9,0 (Leitfähigkeit: 26 - 30 mSi, 20°C) eluiert.

2. Virus-Inaktivierung

Das Eluat wurde mit 1 kg Saccharose und 112 g Glycin pro Liter versetzt, mit Salzsäure auf pH 5,5 eingestellt, auf eine Konzentration von 50.000 IU/ml Urokinase verdünnt und anschließend 10 Stunden bei 60°C erhitzt. Die erhitzte Urokinase-Lösung wurde konzentriert und anschließend sterilfiltriert.

**Patentansprüche**

1. Verfahren zur Aufbereitung von Urokinase durch Adsorption von Urokinase an einen Kationenaustauscher und Desorption der Urokinase und Pasteurisierung einer Lösung dieser Urokinase, dadurch gekennzeichnet, daß zwischen Adsorption und Desorption der Austauscher mit einem Puffer mit einem pH-Wert von 5,4 bis 5,6 gewaschen wird.

2. Verfahren nach Anspruch 1, dadurch gekennzeichnet, daß bei einem pH-Wert von kleiner als 6 pasteurisiert wird.

3. Verfahren nach Anspruch 1, dadurch gekennzeichnet, daß mit einem Puffer mit einer Leitfähigkeit zwischen 5 und 10 mSi gewaschen wird.

4. Verfahren nach Anspruch 1, dadurch gekennzeichnet, daß CM-Cellulose als Kationenaustauscher verwendet wird.

**5.** Verfahren nach Anspruch 1, dadurch gekennzeichnet, daß eine entsalzte Lösung von Urokinase verwendet wird.

**6.** Verfahren nach Anspruch 1, dadurch gekennzeichnet, daß eine mit einem Anionenaustauscher behandelte Lösung von Urokinase verwendet wird.

**7.** Verfahren nach Anspruch 1, dadurch gekennzeichnet, daß in Gegenwart von Saccharose pasteurisiert wird.

**8.** Verfahren nach Anspruch 7, dadurch gekennzeichnet, daß in Gegenwart von Glycin pasteurisiert wird.

**9.** Stabile Urokinase-Präparation, erhältlich nach mindestens einem der Ansprüche 1-8, dadurch gekennzeichnet, daß sie frei von verunreinigenden Proteasen ist und daß sie ein Verhältnis von HMW-UK:LMW-UK aufweist, das dem Verhältnis im verwendeten Ausgangsmaterial entspricht.

**Claims**

**1.** A process for the preparative treatment of urokinase by adsorption of urokinase onto a cation exchanger and desorption of the urokinase and pasteurization of a solution of this urokinase, which comprises washing the exchanger with a buffer with a pH of 5.4 to 5.6 between adsorption and desorption.

**2.** The process as claimed in claim 1, wherein pasteurization is carried out at a pH below 6.

**3.** The process as claimed in claim 1, wherein washing is carried out with a buffer with a conductivity between 5 and 10 mSi.

**4.** The process as claimed in claim 1, wherein CM-cellulose is used as cation exchanger.

**5.** The process as claimed in claim 1, wherein a desalted solution of urokinase is used.

**6.** The process as claimed in claim 1, wherein a solution, which has been treated with an anion exchanger, of urokinase is used.

**7.** The process as claimed in claim 1, wherein pasteurization is carried out in the presence of sucrose.

**8.** The process as claimed in claim 7, wherein pasteurization is carried out in the presence of glycine.

**9.** A stable urokinase preparation obtainable as claimed in at least one of claims 1-8, which is free of contaminating proteases and which has an HMW-UK:LMW-UK ratio which corresponds to the ratio in the starting material used.

**Revendications**

**1.** Procédé de traitement de l'urokinase par adsorption de l'urokinase sur un échangeur de cations et désorption de l'urokinase, et pasteurisation d'une solution de cette urokinase, caractérisé en ce que, entre l'adsorption et la désorption, on lave l'échangeur avec un tampon dont le pH est de 5,4 à 5,6.

**2.** Procédé selon la revendication 1, caractérisé en ce qu'on effectue la pasteurisation à un pH inférieur à 6.

**3.** Procédé selon la revendication 1, caractérisé en ce qu'on effectue le lavage avec un tampon dont la conductibilité est comprise entre 5 et 10 mSi.

**4.** Procédé selon la revendication 1, caractérisé en ce qu'on utilise de la CM-cellulose comme échangeur de cations.

**5.** Procédé selon la revendication 1, caractérisé en ce qu'on utilise une solution déminéralisée d'urokinase.

**6.** Procédé selon la revendication 1, caractérisé en ce qu'on utilise une solution d'urokinase qui a été traitée par un échangeur d'anions.

**7.** Procédé selon la revendication 1, caractérisé en ce qu'on effectue la pasteurisation en présence de saccharose.

**8.** Procédé selon la revendication 7, caractérisé en ce qu'on effectue la pasteurisation en présence de glycine.

**9.** Préparation stable d'urokinase, obtenue selon au moins une des revendications 1 à 8, caractérisée en ce qu'elle est dépourvue de protéases contaminantes et elle présente un rapport de HMW-UK:LMW-UK correspondant au rapport qui existe dans la matière utilisée au départ.